# EUROPEAN PATENT SPECIFICATION

(11) **EP 1 196 593 B1**
(45) Date of publication and mention of the grant of the patent: **25.08.2004**
(21) Application number: 00951284.9
(22) Date of filing: 15.06.2000
(51) Int. Cl.: C12N 15/31, C12N 15/74, C12R 1/46, C07K 14/315, A23C 19/032

(54) **PHAGE-RESISTANT MICROORGANISMS AND GENETIC DETERMINANTS OF PHAGE RESISTANCE**
PHAGENRESISTENTE MIKROORGANISMEN UND GENETISCHE DETERMINANTEN FÜR PHAGENRESISTENZ
MICRO-ORGANISMES RESISTANTS AUX BACTERIOPHAGES ET DETERMINANTS GENETIQUES DE LA RESISTANCE AUX BACTERIOPHAGES

(30) Priority: 27.07.1999 IT MI991654
(43) Date of publication of application: 17.04.2002
(73) Proprietor: ANIDRAL S.R.L., 28100 Novara (IT)
(72) Inventor: MOGNA, Giovanni, I-28100 Novara (IT); STROZZI, Paolo, I-28100 Novara (IT); DI LORENZO, Simona, I-29100 Piacenza (IT); BOTTAZZI, Vittorio Univ.Cattolica del Sacro Cuore, I-29100 Piacenza (IT); CALLEGARI, Maria L. Univ.Cattolic.del Sacro Cuore, I-29100 Piacenza (IT); MORELLI, Lorenzo Univ.Cattolica del Sacro Cuore, I-29100 Piacenza (IT)
(74) Representative: Minoja, Fabrizio, Dr.
(86) International application number: PCT/EP2000/005503
(87) International publication number: WO 2001/007566

(56) References cited:
- WO-A-96/21017
- FR-A- 2 767 831
- O'SULLIVAN T ET AL.: "Structural and functional analysis of pCI65st, a 6.5 kb plasmid from Streptococuus thermophilus NDI-6" MICROBIOLOGY, vol. 145, no. 1, January 1999 (1999-01), pages 127-134, XP000978319
- JANZEN T ET AL.: "Sequencing and characterization of pST1, a cryptic plasmid from Streptococcus thermophilus" FEMS MICROBIOLOGY LETTERS, vol. 74, no. 2-3, 15 August 1992 (1992-08-15), pages 175-180, XP000979636
- ALLISON G E ET AL.: "Phage resistance mechanisms in lactic acid bacteria" INTERNATIONAL DAIRY JOURNAL, vol. 8, no. 3, March 1998 (1998-03), pages 207-226, XP000978412

### SEQUENCE LISTING

<110> ANIDRAL S.r.l.
<120> Phage-resistant microorganisms and gene determinants of phage resistance
<130> 5771M
<140>
<141>
<160> 1
<170> Patent In Ver. 2.1
<210> 1
<211> 1111
<212> DNA
<213> Streptococcus thermophilus
<400> 1
   atggtgaagg taaatttatt acttacatgg aatgtattca gtaatcctgt agctgattta 60
   gatggacttg aaagtgtaga aattgataat aaacagtttc aggttaaggc tggagatgtt 120
   ctatttacta cttcatcaga aactccagaa gaagttggaa tgtcatctat gtggcttgga 180
   aatgcagaca atatctatct taatagcttt tgttttggat atcgaccaac tattgaattt 240
   gataaatatt atctagcggt catgttgagg tctgctccaa ttagaaagaa atttcagtta 300
   cttacacaag gaatttctag atataacatt tcaaagaata aagttatgga aatgtctatt 360
   cctgttccta gcattgaaga acaagaatta cttggagcat ttttcaacaa cctcaatcaa 420
   accatcgctc ttcatcagcg taagttagat ttgttgaaag agcagaaaaa aggcttttta 480
   caaaaaatgt tccctaaaaa tggtgccaaa gttcctgaat tgcgatttgc ggggtttgct 540
   gacgattggg aagagcgtaa gttgggagat atttcctata aggtcaaaga aaaaaataaa 600
   actggtgagt ttacagaaac tttgaccaac tcagcagaat atggaattat taatcaacgt 660
   gattttttta ataaagatat ttctaacgct aagaatctta ctggctatta tgtagttaaa 720
   aacgatgatt ttgtatacaa tcctcgtatt tcaaattttg ctccggttgg accaattaaa 780
   cgtaataaat taggtagaac tggcgtaatg tcaccactat attatgtttt ccgtacacat 840
   gatatcgata aaaattacct tgagaagtat tttgatactg tctactggca tcgattcatg 900
   aaactaaacg gtgactcagg agtacgtgcc gatcgttttg caattaagga ctctgtcttt 960
   gttgaaatgc caattcctta tccaacgatt gaagaacaag aaaaaatagg ttcattcttc 1020
   aaacagttag acgatactat cgctcttcat cagcgtaagt tagatttgtt gaaagagcag 1080
   aaaaaaggct ttttacaaaa gatgtttgtt t                               1111

## Description

The present invention relates to novel bacterial strains, the plasmids derived thereof, a gene sequence included in the plasmids, encoding a protein of the phage resistance system, and a method to confer such a resistance to microorganism cultures.

### INVENTION BACKGROUND

Lactic bacteria have a fundamental role in the manufacturing process of milk derivatives, particularly of the fermented milks and cheeses.

Their action takes place in the first phases of cheese transformation, inducing some modifications in the milk and/or in the curd, depending on the production rate and amount of lactic acid obtained from lactose fermentation.

The acidifying capacity and total enzyme activity of the lactic bacteria starter culture, used in specific dairy manufacturing steps, are fundamental technological parameters, which determine the organoleptic and structural characteristics of the finished product.

These metabolic properties are typical of the different species of lactic bacteria and depend, quantitatively, in different ways, on the number and the degree of vitality of the lactic bacteria in the culture and on their multiplication rate in milk and subsequently in curd.

A delay or even worse, a block in the growth starter, can cause serious manufacturing problems, impairing the industrial process as a whole.

A more frequent cause of slowed down or completely blocked bacterial replication is due to the presence of bacteriophages, viruses able to replicate inside a bacterial cell. Bacteriophages, or phages, are able to recognize and specifically attack the host cell and, in the lytic cycle, to totally destroy it, releasing dozens or hundreds of other virulent phages able to attack other sensitive bacterial cells.

The event, first described in 1935, constitutes to date, one of the most serious problems affecting the cheese industry because when the phage infection starts, the possibility arises that production cannot be completed thus determining huge economic loss.

The most promising results for overcoming this problem have been achieved using bacterial cultures comprised of:
a) strains with different lysotype (phage sensitivity) used in rotation;
b) phage-resistant strains.

The first solution, at the moment the most adopted by the starter-producing companies, involves considerable organization efforts from culture providers and users, and, in any case, does not permit a complete standardization of the finished product. This is because it is almost impossible to obtain, to isolate, and to produce bacterial strains with identical technological characteristics but with a different lysotype.

The second solution can be obtained by different mechanisms.

The phage-resistant strains arise spontaneously in sensitive populations following phage attack.

The phage-resistance mechanisms outlined in these strains can be grouped into three categories:
1. block of 'adsorption on the bacterial wall and subsequent block of the phage DNA entry in the cytoplasm;
2. phage DNA restriction (enzyme cleavage) upon entry inside the bacterial cell;
3. interference with the phage DNA duplication mechanisms upon its entrance inside the bacterial cell (abortive infection).

However, spontaneous phage-resistant mutants are generally characterized by a scarce technological aptitude, making them unsuitable for industrial use (King W.R. et al., Appl. Environ. Microbiol., 1983, 45, 1481-1485; Steenson L.R. et al., Dairy Sci., 1986 69, 2227-2236).

Recently inventions have been described which deal with the problem of obtaining phage-resistant strains with high technological properties by means of genetic engineering aimed at introducing genes encoding one or more of the above listed mechanisms in starter strains (patents US-5,824,523 and US-5,538,864).

This approach presents some drawbacks from the industrial point of view because these microorganisms and the foods obtained therefrom are included, at least in the European Community, in the "Novel Foods" category, governed by the EC regulation N°. 258/97 of January, 27, 1997.

The possibility to obtain phage-resistant culture strains by using natural gene transfer techniques is of particular interest.

For this purpose it is necessary to select genetic elements able to recombine and mobilize in vivo and to contemporarily confer elevated phage resistance levels.

O'Sullivan et al. (Microbiology, vol. 145, no. 1, 1999, pp. 127-134), report the sequencing and characteriztion of the 6.5 kb cryptic plasmid pCI65st from *Streptococcus Thermophilus* NDI-6, a strain. isolated from the Indian fermented milk dahl. This plasmid confers heat-shock resistance as well as bacteriophage-lysis resistance.

The encoded proteins are similar to the HsdS or specificity proteins from the Type I restriction-modification systems.

### DESCRIPTION OF THE INVENTION

The authors of the present application isolated novel Streptococcus *thermophilus* strains showing phage-resistance. These strains were taxonomically, technologically, and genetically characterized. Furthermore, gene elements responsible for the phage resistance were isolated and characterized.

The parental strain, called TO03, was deposited at the BCGM™ /lmg Bacteria Collection (Gent-Belgio) at N. P-18384, whilst the corresponding phage-resistant mutant, termed B39, was deposited at the same collection at N. P-18383. Both strains represent the first aspect of the invention. These strains contain the gene information conferring phage resistance, but only in the B39 strain, in which the two plasmids - otherwise contained as two distinct molecules in the wild type - are genetically recombined, phage resistance is observed. The B39 phage-resistant phenotype has, for its use in the dairy-milk field, the same properties as the parental strain; in particular, the acidifying rate in milk is the same as that of the TO03 strain, allowing the use of the B39 strain as starter culture in the same dairy processes in which the parental strain is used.

The TOO3 strain has two plasmids, termed pCRB33 and pCRB63, in which two ORFs (Open Reading Frames) were found presenting high homology with the "s" subunits, known to be involved in the type I restriction and modification mechanisms. In the TO03 strains these two ORF are incomplete and thus inactive. The above plasmids can recombine, creating the pCRB96 plasmid, in which the incomplete and inactive ORFs give, upon recombination, a complete and active "s" subunit.

The pCRB33, pCRB63, and pCRB96 plasmids are further embodiments of the invention.

Such plasmids are described in the following example 1. In particular, the complete restriction map is provided for each plasmid.

In another aspect, the invention relates to the gene determinant responsible for conferring phage resistance. This determinant corresponds to the ORF of pCRB96 plasmid, encoding the above mentioned "s" subunit, whose sequence is reported in SEQ. ID N. 1. Such a protein, in the type I restriction and modification systems, confers specificity to the restriction enzyme and methylase. The subunit alone is not able to confer phage resistance; actually transferring it to a heterologous host does not automatically determine phenotype change, the presence of gene encoding the two involved enzymes being necessary. The introduction in a host containing a complete type I R/M of a heterologous s subunit, may result in an enhanced phage resistance, comparable to a complete R/M system. In fact, the s subunit alone can change the system specificity, without inhibiting the pre-existing one. Resistance results from the sum of the effects of the two subunits.

The gene determinant can be inserted in any suitable plasmid using conventional techniques (for example as described in Maniatis, T. et al., Molecular Cloning: A Laboratory Manual, Cold Spring Harbor Laboratory, 1982).

A plasmid containing the gene determinant for phage resistance described herein is another object of the invention.

The non-conjugative plasmids, such as pCRB96, can be used in conjunction with other plasmids able to mediate their transfer. Therefore, in another aspect, the invention relates to the use of plasmids containing the gene determinant of phage resistance described herein, alone or in combination with a conjugative plasmid, to confer phage resistance to bacteria.

Another aspect of the invention relates to a host microorganism in which the plasmid containing the phage resistance gene determinant, in accordance with the invention, is able to replicate. The plasmid can be introduced by conventional techniques, such as conjugative transfer and transformation.

Besides *Streptococcus thermophilus,* the host can be *Bacillus subtilis* or *Escherichia coli,* or preferably Lactococcus lactis. The introduction by transformation of the s subunit in a heterologous host of genera and species other than *Streptococcus thermophilus,* but endowed with the same industrial interest, can be accomplished by vectors suitable for the host itself. The microorganisms containing the plasmid of the invention are particularly useful in the production of milk derivative such as fermented milks and cheeses. They are used as starter cultures, comprised of single or multiple strains.

### DETAILED DESCRIPTION OF THE INVENTION

The Streptococcus thermophilus TO03 strain, used as starter culture in the fresh and pasta filata cheeses, proved to be sensitive to the lytic bacteriophage SST3 attack.

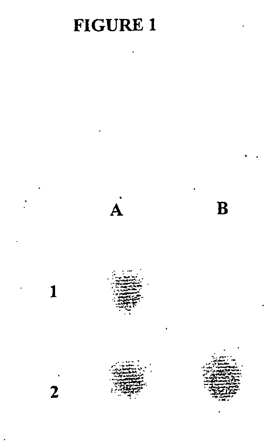

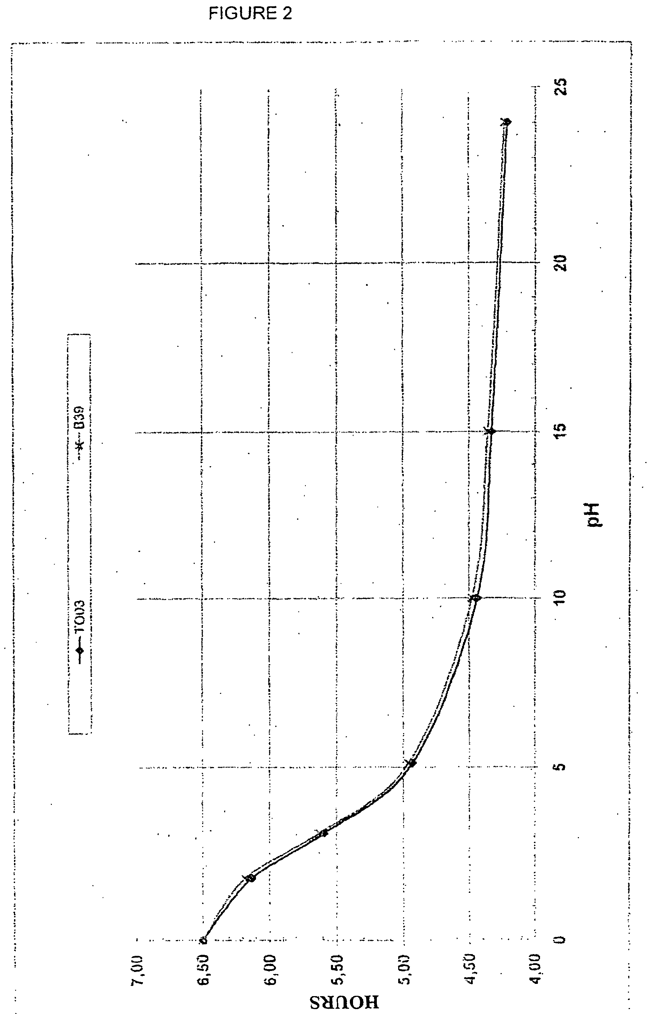

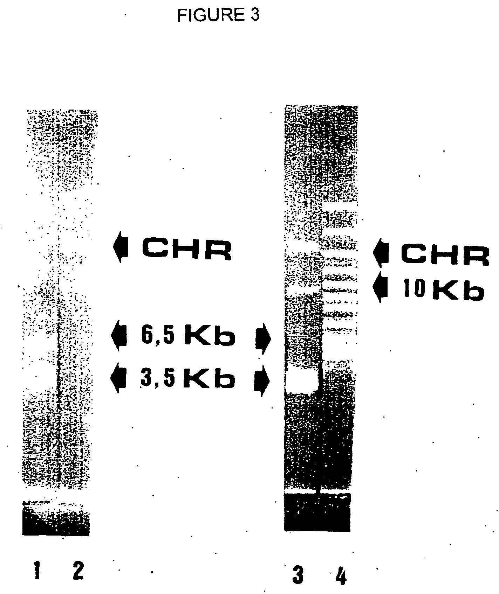

The TO03 strain was characterized by:
- taxonomy, obtained by hybridization with a 23 S rRNA specific probe according to Ehmann et al., 1992 (Fig. 1);
- sugar fermentation profile, obtained by API tunnels, which was positive to glucose, fructose, lactose and sucrose;
- acidifying capacity in sterile skim milk at 37°C, as determined by continuous pH detection (Fig. 2);
- two extra-chromosomal DNA or plasmids, 3,3 and 6,3 kb in size, named pCRB33 and pCRB63, respectively (Fig. 3).

The TO03 strain was attacked by the SST3 lytic phage, with a phage/bacterial cell ratio of 1:10 (m.o.i. 0,1). The surviving cells were plated on M17 agar medium. The plates were then incubated under anaerobiosis at 42°C overnight. This procedure allowed the identification of 40 colonies of the original bacterial population, constituted of 10⁹ CFU/ml. The isolated colonies were further assayed for phage resistance.

Also the obtained phage-resistant isolates were characterized by their sugar fermentation profile, their acidifying capacity in milk and extra-chromosomal DNA content.

Thus the resistant phage isolates could be divided into two groups:
- Group R1 comprising isolates containing the two plasmids, pCRB33, pCRB63 and the additional pCRB96 plasmid.
- Group R2 comprising isolates containing the pCRB33 and the additional pCRB96 plasmid.

All isolates were taxonomically identical to the parental strain, showed the same sugar fermentation profile, but grew more slowly in milk, making them of low technological usefulness.

An isolate of the R2 group was successively grown in liquid M17 at 30°C, a temperature lower than the optimal one. The bacterial population obtained in these conditions was plated on agar medium and further subjected to phage attack.

The CFU, that proved to be stably phage-resistant, about 50% of the isolates, were thus again tested in order to evaluate their acidifying rate in milk. Surprisingly, in one of these colonies, termed B39, an acidifying rate similar to that of the parental strain was observed (Fig. 2).

The plasmid profile analysis of this strain demonstrated the presence of the 9,6 kb plasmid, termed pCRB96, alone.

In order to study the involvement of the pCRB96 plasmid in phage resistance, we proceeded to the curing of the plasmid itself. The obtained clone, termed C48, was free of plasmids and SST3 phage sensitive. In order to obtain a further confirmation of the role of this plasmid, we proceeded to reintroduce it by conjugation into the C48 clone. However the C48 clone had the same phenotypic characters as the B39 strain, making discrimination between donors and recipients after the conjugation impossible. To overcome this drawback we selected a fusidic acid-resistant clone, starting from C48.

The C48 strain was UV irradiated and the surviving cells were seeded in M17 agar medium, containing fusidic acid. The aim was to select a mutant to this type of antibiotic in order to use it as a recipient selector after conjugation.

The obtained clone was named TO60.

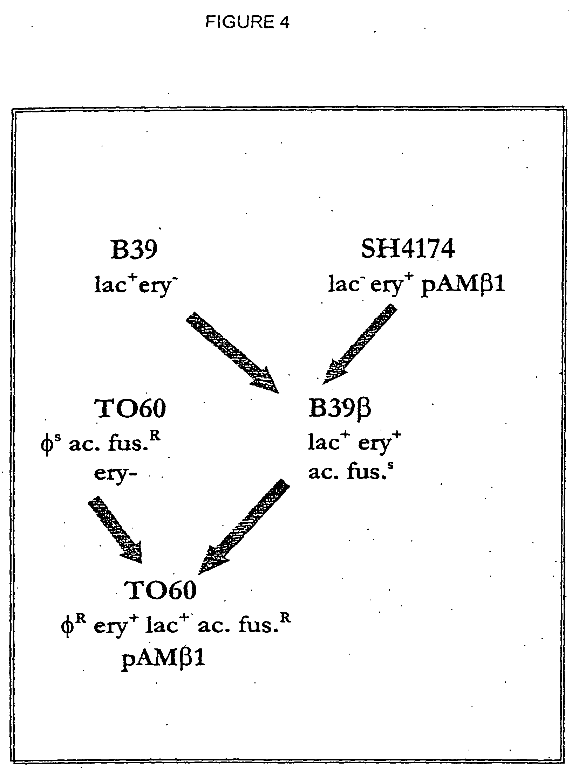

The UV rays did not alter the clone SST3 phage sensitivity. Because the pCRB96 plasmid is conjugative, it was necessary to perform a co-transfer of the plasmid.itself mediated by pAMβ₁. This is a conjugative plasmid and it encodes erithromycin resistance. The subsequent steps, aimed at obtaining the plasmid transfer, are illustrated in Fig. 4 and can be summarized as follows:
- the pAMβ₁ plasmid was transferred from the donor strain *Lactobacillus lactis* subsp. *lactis* SH41.74, to the B39 clone by a first conjugation. The colonies of this donor strain were counted in M17 plates containing glucose and erithromycin, and incubated at 30°C under anaerobic conditions. The trans-conjugating colonies were selected on M17 plates containing lactose and erithromycin and incubated at 42°C in anaerobic conditions;
- in a second conjugation event we used the B39 clone (containing pAMβ₁,) as the donor strain and the TO60 clone as the recipient strain. In this case, the expected trans-conjugants were resistant to erithromycin and also to fusidic acid. All the colonies with these characteristics were assayed for phage resistance.

Some of them (11 over a total of 350 assayed colonies) proved to be phage resistant. The plasmid content of these clones demonstrated the contemporary presence of the pAMβ₁ and the pCRB96 plasmids.

The curing experiments and the pCRB96 plasmid transfer have thus demonstrated that the phage resistance of the clones isolated starting from the TO03 strain was linked to the presence of such plasmid.

The phage-resistant clones did not show full resistance against the SST3 phage, but the number of the phage plaques obtained on the plate was reduced compared to the PFU number obtained with the TO03 strain of at least 2 log. In order to identify the type of phage resistance involved, we performed cross-hybridizations between the SST3 phage propagated in the TO03 strain, and the same phage propagated in the B39 strain.

For convenience the latter phage was called SST39.

As demonstrated in table 1 the phage titrated on the sensitive strain, showed a higher titre than that obtained when the host strain was B39. On the other hand the phage multiplied on the B39 strain produced the same PFU/ml when titrated on the two strains.

**Table 1**

| Host strain | SST3 phage | SST39 phage |
|---|---|---|
| | PFU/ml | PFU/ml |
| TO03 | 2x10⁸ | 3x10⁷ |
| B39 | 3x10⁶ | 3x10⁷ |
| C48 | 2x10⁸ | 3x10⁷ |

Table 2 outlines the results of the titrations obtained on the sensitive strains and on the strain resistant to the SST39 propagated on TO03 and B39, respectively. It is possible to observe that the SST39 phage lost its ability to attack with high efficiency the phage resistant strain upon propagation on the TO03 strain. This behavior is typical of restriction and modification systems. We thus attributed a role in an R/M system to the pCRB96 plasmid.

**Table 2**

| Host strain | SST39/TO03 phage | SST39/B39 phage |
|---|---|---|
| | PFU/ml | PFU/ml |
| TO03 | 2x10⁷ | 3x10⁸ |
| B39 | 3x10⁵ | 3x10⁸ |
| C48 | 2x10⁷ | 3x10⁸ |

### Plasmid DNA analysis

The restriction map analysis of the pCRB33, pCRB63, and pCRB96 plasmids suggested that the latter could be the result of the integration of the two plasmids originally located in the TO03 phage-resistant strain. The first confirmations were obtained in DNA/DNA hybridization experiments. With the latter method, in fact, signals were obtained when the pCRB96 plasmid was hybridized to probes comprised of pCRB33 and pCRB63 fragments.

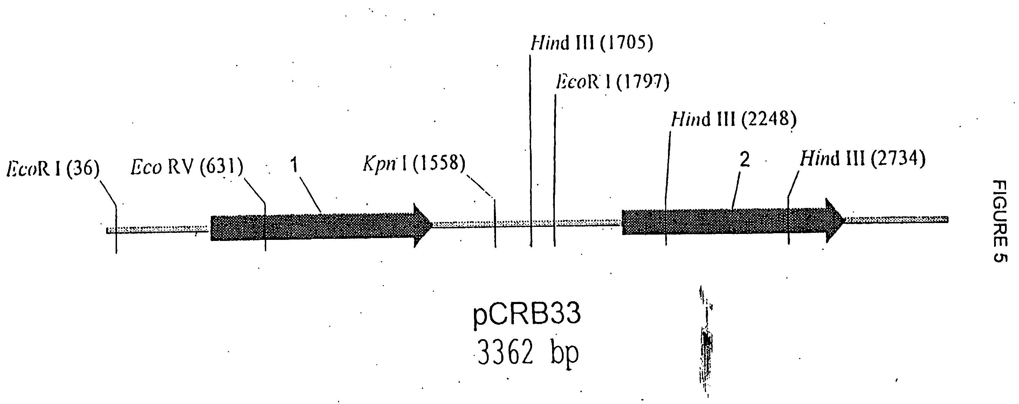

In order to obtain further evidence of the integration event we conducted cloning and sequencing of the pCRB33 plasmid. The plasmid graphic representation is shown in Fig. 5. From the sequence analysis we could localize two complete ORFs, indicated in fig. 5 ORF1 and ORF2, respectively.

The ORF2 showed a high homology (87%) with respect to the sequence of the RepA protein, located on the pST1 plasmid of the Streptococcus thermophilus ST strain (deposit number GENEBANK X65856). A termination region was found downstream from the coding region, constituted of repeated sequences with 86% homology compared to that of the above mentioned RepA. The ORF1 shows homology in some portions to many s subunits of type I restriction and modification system.

The higher homologies were found in a 133 bp region whose sequence contains one of the two conserved motifs from the s subunits. The same homology to the s subunits was found also in a region of 153 bp outside the ORF1 and 473 bp distant from the end of the first region. These two regions can be considered two repeated direct sequences. We named the first 133 bp sequence DR1, and the second 153 bp sequence DR2.

Using a primer set designed on the sequence of the two DR found in pCRB33, the pCRB96 plasmid was amplified by PCR. The amplification product was made up of two fragments of 3,3 and 6,3 kb, respectively. These results induced us to hypothesize that the two DRs would be located in the integration region.

In summary pCRB33 contains a gene encoding a protein responsible for replication and probably two DR involved in the integration event.

We thus cloned and determined the nucleotide sequence of the pCRB63 plasmid. The sequence analysis did not show any homology to the known genes encoding for phage resistance apart from the ORF1, whose sequence showed a region endowed with high homology with respect to the different s subunit of type I restriction and modification systems, exactly as previously demonstrated for the pCRB33 plasmid ORF1. The revealed homology also in this case concerned the conserved motifs of the s subunits. Also in the pCRB63 plasmid are present the two DR, exactly as for pCRB33.

The pCRB96 plasmid was thus fully sequenced and resulted to be a co-integration product of the pCRB33 and pCRB63 plasmids.

The two regions in which the integration takes place are those delimited by the two DRs, whilst the region between them is where the two plasmids are cut and joined together. In fact, in pCRB96 there are 2 regions in which DR1 and DR2 are present. The pCRB33 DR1 is, in this case, associated with the pCRB63 DR2, whilst the smaller plasmid DR2 is associated with the pCRB63 DR1.

The ORF1 (Fig. 2), with high homology to the s subunits of type 1 R/M systems, in particular to the s subunit isolated from *Lactobacillus lactis.* IL-1403 and the s subunit of *L*/dl of *Lactococcus lactis* subsp. *cremoris* (deposit number GENEBANK AF 034786 and U90222) which are 55% homologous, was located in one of the integration regions. In the case of pCRB96, the sequence, homology, and phenotype demonstrate that the s subunit encoding gene is complete and functional.

On the contrary, pCRB33 and pCRB63 contain ORFs with homology to genes encoding the s subunit of type I R/M systems, but they are incomplete and thus not functional. Only pCRB96, by means of integration, has the functional gene, whose sequence is the sum of pCRB33 ORF1 and pCRB63 ORF1 portions. The sequence of the entire pCRB96 s subunit is reported in Seq ID N°. 1.

### BRIEF DESCRIPTION OF THE FIGURES

**Figure 1:** Taxonomic identification of the TO03 and B39 strains
   Probe used: CATGCCTTCGCTTACGCT
   Probe and hybridization protocol according to Ehrmann et al. (1992) "Species-specific oligonucleotide probe for the identification of *Streptococcus thermophilus",* Systematic and Applied Microbiology, **15**, 453-455.
   Hybridization results:
   A1: Model strain of the species *Streptococcus thermophilus* DSM 20617 (positive control);
   A2: DNA extracted from *Lactobacillus helveticus* ATCC 15009 (negative control);
   B1: DNA extracted from *Streptococcus thermophilus* B39;
   B2: DNA extracted from *Streptococcus thermophilus* TO03.
      Positive signals were obtained from the reference strain DSM 20617 and from the TO03 and B39 strains under investigation, thus confirming to be *Streptococcus thermophilus* species.
**Figure 2:** Acidification curve: 1% inoculum, sterile skim milk, 37°C.
**Figure 3:** Plasmid profile of the TO03 (well 2) and B39 (well 4) strains.
**Figure 4:** Scheme of the conjugations performed in order to co-transfer pAMβ1 and pCRB96 plasmids.
**Figure 5:** Schematic representation of pCRB33.
   ORF1: ORF located from nt 411 to nt 1308, corresponding to 299 amino acids, of the appended pCRB33 nucleotide sequence. This ORF shows homology to different subunits of type I restriction systems.
   ORF2: ORF located from nt 2070 to nt 2960. This ORF shows 87% homology to the pST1 RepA (acc. num. X65856).

The following examples illustrate the invention in further detail.

### EXAMPLE 1

### Characterization of the pCRB33, pCRB63, and pCRB96 plasmids.

The following tables report the restriction profiles of the pCRB33, pCRB63, and pCRB96 plasmids.

**Table 3:**

| pCRB33 plasmid, 3375 base pairs: | | | |
|---|---|---|---|
| Enzyme name | N°. cuts | Site positions | Recognition sequence |
| Accl | | 1233 | gt/mkac |
| Alul | | 134 138 161 466 622 1137 1670 1714 2259 2421 2745 2932 2982 | ag/ct |
| Asel | | 2055 | at/taat |
| Asnl | | 2055 | at/taat |
| Avall | | 2199 2213 2227 | g/gwcc |
| Ddel | | 157 1144 1215 1671 1695 2159 2203 2255 | c/tnag |
| Dpnl | | 9852253 | ga/tc |
| Dral | | 2828 3024 | ttt/aaa |
| EcoRl | | 39 1804 | g/aattc |
| EcoRV | | 636 | gat/atc |
| Fokl | | 259 | ggatg |
| Haelll | | 2157 2173 3092 3275 | gg/cc |
| Hindlll | | 1712 2257 2743 | a/agctt |
| Hinfl | | 1223 1697 2006 2102 2341 | g/antc |
| Hpall | | 2022174 | c/cgg |
| Kpnl | | 1565 | ggtac/c |
| Mael | | 135 139 669 733 784 1877 2848 2854 3084 3269 3277 | c/tag |
| Maell | | 1081 1201 1468 2887 3049 3174 3306 | a/cgt |
| Maelll | 8 | 712 1082 1177 1752 2499 2872 3045 3307 | /gtnac |
| Mbol | 2 | 9832251 | /gatc |
| Msel | 23 | 8 167 237 243 287 335 358 518 616 1095 1329 1904 1996 2055 2195 2378 2447 2636 2741 2827 2965 3023 3180 | t/taa |
| Notl | 1 | 3090 | gc/ggccgc |
| Pstl | 1 | 3101 | ctgca/g |
| Pvull | 1 | 2421 | cag/ctg |
| Sau3Al | 2 | 983 2251 | /gatc |
| Sau96l | 3 | 2199 2213 2227 | g/gncc |
| Spel | 2 | 1876 3083 | a/ctagt |
| Taql | 12 | 1 632 638 763 1254 1735 1849 2004 2111 2985 3104 3372 | t/cga |
| Xbal | 1 | 732 | t/ctaga |

wherein:
**r** = a or g; **k** = g or t; **h** = a or c or t; **d** = a or g or t; **y** = c or t; **s** = c or g; **b** = c or g or t; n = a or c or g or t; **m** = a or c; **w** = a or t; **v** = a or c or g.
The following endonucleases did not cleave the pCRB33 sequence:
Apal, Ava I, **BamHl,** Bcll, Bgll, Cfol, Clal, Haell, Hincll, Hindll, Hpal, Ncol, Pvul, **Sacl**, Sadl, Sall, **Smal,** Sohl, **Xhol,** Xmal.

**Table 4:**

| pCRB63 plasmid, 6148 base pairs: | | | |
|---|---|---|---|
| Enzym e name | N°. cuts | Site positions | Recognition sequence |
| Accl | 4 | 165 312 2451 4102 | gt/mkac |
| Alul | 20 | 692 850 1358 1487 1518 1965 2264 2270 2471 2625 2913 3155 3667 3717 3744 3793 4038 4270 4648 5530 | ag/ct |
| Asel | 2 | 81 5115 | at/taat |
| Asnl | 2 | 81 5115 | at/taat |
| Aval | 1 | 2702 | c/ycgrg |
| Avail | 2 | 2004368 | g/gwcc |
| Cfol | 8 | 1203 1897 2046 2609 2909 4097 4442 4483 | gcg/c |
| Clal | 2 | 276 322 | at/cgat |
| Ddel | 11 | 61 120 346 1930 2130 3953 4251 4276 4581 5002 5904 | c/tnag |
| Dpnl | 7 | 363 1029 1067 4890 5032 5718 5917 | ga/tc |
| Dral | 5 | 922 2773 4304 5261 5483 | ttt/aaa |
| EcoRl | 2 | 1569 3413 | g/aattc |
| EcoRV | 2 | 2742510 | gat/atc |
| Fokl | 6 | 3875 3905 3911 4961 5500 5565 | ggatg |
| Haell | 2 | 1898 4098 | rgcgc/y |
| Haelll | 3 | 2189 3001 5891 | gg/cc |
| Hincll | 2 | 2452 3706 | gty/rac |
| Hindll | 2 | 24523706 | gty/rac |
| Hindlll | 1 | 2623 | a/agctt |
| Hinfll | 12 | 124 324 344 380 612 1434 2146 2453 3955 4255 5355 5448 | g/antc |
| Hpall | 3 | 194 3396 5812 | c/cgg |
| Kpnl | 1 | 580 | ggtac/c |
| Mael | 16 | 609 1580 1916 2271 2540 2721 2910 3597 4079 4122 4184 4622 4985 5231 5272 5945 | c/tag |
| Maell | 19 | 88 211 355 877 1529 2092 2318 2545 2827 2877 3283 3349 3367 3624 3686 3887 3987 4192 6016 | a/cgt |
| Maelll | 12 | 240 341 1094 1260 2011 2273 2878 3350 3590 4056 4773 5379 | /gtnac |
| Mbol | 7 | 361 1027 1065 4888 5030 5716 5915 | /gatc |
| Msel | 44 | 81 99 147 207 375 921 1062 1151 1481 2219 2410 2711 2772 3020 3092 3204 3221 3443 3491 3544 3644 3694 3750 3825 3937 3993 4003 4141 4229 4303 4407 4530 4550 4750 4847 5015 5115 5260 5455 5482 5548 5626 5630 5666 | t/taa |
| Pvul | 1 | 364 | cgat/cg |
| Sacl | 1 | 3795 | gagct/c |
| Sacll | 1 | 2020 | ccgc/gg |
| Sall | 1 | 2450 | g/tcgac |
| Sau3Al | 7 | 361 1027 1065 4888 5030 5716 5915 | /gatc |
| Sau96l | 3 | 200 4368 5889 | g/gncc |
| Spel | 1 | 4183 | a/ctagt |
| Sphl | 1 | 5743 | gcatg/c |
| Taql | 13 | 276 322 2377 2451 2465 2473 2620 2808 2866 3377 3726 4666 5921 | t/cga |
| Xbal | 4 | 608 2720 4078 4121 | t/ctaga |

**r** = a or g; **k** = g or t; **h** = a or c or t; **d** = a or g or t; **y** = c or t; **s** = c or g; **b** = c or g or t; **n** = a or c or g or t; **m** = a or c: **w** = a or t; **v** = a or c or g.
The following endonucleases did not cleave the pCRB33 sequence:
Apal, **BamHl**, Bcll, Bgll, Hpal, Ncol, **Pstl**, Pvull, **Smal, Xhol**, Xmal.

**Table 5:**

| pCRB96 plasmid, 9515 base pairs: | | | |
|---|---|---|---|
| Enzyme name | N°. cuts | Site positions | Recognition sequence |
| Accl | 5 | 1150 1297 3436 5087 7381 | gt/mkac |
| Alul | 33 | 134 138 161 466 622 1677 1835 2343 2472 2503 2950 3249 3255 3456 3610 3898 4140 4652 4702 4729 4778 5023 5255 5633 6515 7284 7815 7859 8402 8564 8888 9072 9122 | ag/ct |
| Asel | 3 | 1066 6100 8200 | at/taat |
| Asnl | 3 | 1066 6100 8200 | at/taat |
| Aval | 1 | 3687 | c/ycgrg |
| Avall | 5 | 1185 5353 8342 8356 8370 | g/gwcc |
| Cfol | 8 | 2188 2882 3031 3594 3894 5082 5427 5468 | gcg/c |
| Clal | 2 | 1261 1307 | at/cgat |
| Ddel | 18 | 157 1046 1105 1331 2915 3115 4938 5236 5261 5566 5987 6889 7363 7816 7840 8302 8346 8398 | c/tnag |
| Dpnl | 9 | 1348 2014 2052 5875 6017 6703 6902 7132 8396 | ga/tc |
| Drat | 7 | 1907 3758 5289 6246 6468 8969 9164 | ttt/aaa |
| EcoRl | 4 | 39 2554 4398 7949 | g/aattc |
| EcoRV | 3 | 636 1259 3495 | gat/atc |
| Fokl | 7 | 259 4860 4890 4896 5946 6485 6550 | ggatg |
| Haell | 2 | 2883 5083 | rgcgc/y |
| Haelll | 7 | 3174 3986 6876 8300 8316 9232 9415 | gg/cc |
| Hincll | 2 | 3437 4691 | gty/rac |
| Hindll | 2 | 3437 4691 | gty/rac |
| Hindlll | 4 | 3608 7857 8400 8886 | a/agctt |
| Hinfll | 17 | 1109 1309 1329 1365 1597 2419 3131 3438 4940 5240 6340 6433 7371 7842 8151 8245 8484 | g/antc |
| Hpall | 5 | 202 1179 4381 6797 8317 | c/cgg |
| Kpnl | 2 | 1565 7710 | ggtac/c |
| Mael | 27 | 135 139 669 733 784 1594 2565 2901 3256 3525 3706 3895 4582 5064 5107 5169 5607 5970 6216 6257 6930 8022 8988 8994 9224 94099417 | c/tag |
| Maell | 26 | 1073 1196 1340 1862 2514 3077 3303 3530 3812 3862 4268 4334 4352 4609 4671 4872 4972 5177 7001 7228 7349 7614 9027 9189 9314 9446 | a/cgt |
| Maelll | 21 | 712 1225 1326 2079 2245 2996 3258 3863 4335 4575 5041 5758 6364 7229 7325 7897 8415 8642 9012 9185 9447 | /gtnac |
| Mbol | 9 | 1346 2012 2050 5873 6015 6701 6900 7130 8394 | /gatc |
| Msel | 68 | 8 167 237 243 287 335 358 518 616 1066 1084 1132 1192 1360 1906 2047 2136 2466 3204 3395 3696 3757 4005 4077 4189 4206 4428 4476 4529 4629 4679 4735 4810 4922 4978 4988 5126 5214 5288 5392 5515 5535 5735 5832 6000 6100 6245 6440 6467 6533 6611 6615 6651 7242 7291 7475 8049 8141 8200 8338 8521 8590 8779 8884 8968 9105 9163 9320 | t/taa |
| Notl | 1 | 9230 | gc/ggccgc |
| Pstl | 1 | 9241 | ctgca/g |
| Pvul | 1 | 1349 | cgat/cg |
| Pvull | 1 | 8564 | cag/ctg |
| Sacl | 1 | 4780 | gagct/c |
| Sacll | 1 | 3005 | ccgc/gg |
| Sall | 1 | 3435 | g/tcgac |
| Sau3Al | 9 | 1346 2012 2050 5873 6015 6701 6900 7130 8394 | /gatc |
| Sau96l | 6 | 1185 5353 6874 8342 8356 8370 | g/gncc |
| Spel | 3 | 5168 8021 9223 | a/ctagt |
| Sphl | 1 | 6728 | gcatg/c |
| Taql | 23 | 1 638 1261 1307 3362 3436 3450 3458 3605 3793 3851 4362 4711 5651 6906 7402 7880 7994 8149 8254 9125 9244 9512 | t/cga |
| Xbal | 5 | 732 1593 3705 5063 5106 | t/ctaga |

**r** = a or g; **k** = g or t; **h** = a or c or t; **d** = a or g or t; **y** = c or t: **s** = c or g; **b** = c or g or t; **n** = a or c or g or t; **m** = a or c; **w** = a or t; **v** = a or c or g.
The following endonucleases did not cleave the pCRB96 sequence:
Apal, **BamHl**, Bcll, Bgll, Hpal, Ncol, **Smal, Xhol,** Xmal.

### EXAMPLE 2

### Conjugative transfers

1. In the first conjugation cycle, the cultures of the donor strain *Lactobacillus lactis* SH4174 containing the pAMβ1 plasmid encoding erithromycin resistance, and cultures of the recipient strains *Streptococcus thermophilus* B39, containing the pCRB96 plasmid, are grown.
2. In the second conjugation cycle, the donor and recipient strain cultures of *Streptococcus thermophilus* B39 (pAMβ1) containing the pAMβ1 plasmid and the pCRB96 plasmid, and of *Streptococcus thermophilus* TO60, plasmid-free and resistant to fusidic acid, respectively, are grown.

### Procedure

Equal volumes are taken from both cultures and mixed.

From this mix 0,2 ml are taken, placed on a Petri dish containing M17 medium without any selection agent, uniformly plated, and incubated from 6 to 30 hours.

The bacterial cells grown on this medium are harvested with 1 ml of saline and then appropriate decimal dilutions, in culture media (see table) suitable for selecting donor and recipient strains, and possible trans-conjugants present in the conjugation mix, were seeded on plate.

**Table 6:**

| First conjugation cycle. | | |
|---|---|---|
| | STRAINS | SELECTION |
| DONORS | SH4174 | 30°C, 50 µg/m) erithromycin, glucosate M17 medium |
| RECIPIENT S | B39 (pCRB96) | 42°C, lactosate M17 medium |
| TRANSCONJUGAT ES | B39 (pCRB96-pAMβ1) | 42°C, lactosate M17 medium, 10 µg/ml erithromycin |

**Table 7:**

| Second conjugation cycle. | | |
|---|---|---|
| | STRAINS | SELECTION |
| DONORS | B39 (pCRB96-pAMβ1) | 42°C, lactosate M17 medium, 10 µg/ml erithromycin |
| RECIPIENTS | TO60 | 42°C, lactosate M17 medium, 10 µg/ml fusidic acid |
| TRANSCONJUGATES | TO60 (pCRB96-pAMβ1) | 42°C, lactosate M17 medium, 10 µg/ml erithromycin, 10 µg/ml fusidic acid |

Two types of trans-conjugants are expected from the second conjugation cycle:
one containing only the conjugative pAMβ1 plasmid and one containing both pCRB96 and pAMβ1 plasmids.

### Results

**Table 8:**

| First conjugation cycle. | | |
|---|---|---|
| | STRAINS | Colony Forming Units |
| DONORS | SH4174 | 10⁷ |
| RECIPIENTS | B39 (pCRB96) | 10⁹ |
| TRANSCONJUGATES | B39 (pCRB96-pAMβ1) | 1000 |

**Table 9:**

| Second conjugation cycle. | | |
|---|---|---|
| | STRAINS | Colony Forming Units |
| DONORS | B39 (pCRB96-pAMβ1) | 10⁸ |
| RECIPIENTS | TO60 | 10⁸ |
| TRANSCONJUGANTS | TO60 (pAMβ1) TO60 (pCRB96-pAMβ1) | 10⁴ (pAMβ1) 50 (pCRB96-pAMβ1) |

In the second conjugation cycle only 50 Colony Forming Units were subjected to co-mobilization of the pCRB96 plasmid by the pAMβ1 plasmid.

The phage resistance levels of the TO60 trans-conjugants (pCRB96-pAMβ1) were identical to those of B39.

The phage sensitivity levels of TO60 and TO60 (pAMβ1) were identical to those of TO03.

## Claims

1. *Streptococcus thermophilus* strains deposited at BCCM/LMG (Gent/Belgium) P-18383 and P-18384.

2. Nucleic acid molecule having the sequence Seq. ID N°. 1.

3. Plasmid containing the nucleic acid molecule of claim 2.

4. Plasmid obtainable from a culture of *Streptococcus thermophilus* strain N. P-18384 according to claim 1, having 3375 base pairs and a single restriction site for each Accl, Asel, Asnl, EcoRV, Fokl, Notl, Pstl, Pvull, Xbal nuclease.

5. Plasmid obtainable from a culture of *Streptococcus thermophilus* strain N. P-18384 according to claim 1, having 6184 base pairs and a single restriction site for each Aval, Hindlll, Kpnl, Pvul, Sacl, Sacll, Sall, Spel, Sphl nuclease.

6. Plasmid obtainable from a culture of *Streptococcus thermophilus* strain N. P-18383 according to claim 1, having 9515 base pairs and a single restriction site for each Aval, Notl, Pstl, Pvul, Pvull, Sacl, Sacll, Sall, Sphl nuclease.

7. Microorganism containing the plasmid of claims 3-6.

8. Microorganism according to claim 7, selected from *Streptococcus thermophilus, Lactobacillus lactis, Bacillus subtilis, Escherichia coli, Lc. lactis subsp. lactis, Lc. lactis subsp. diacetylactis, Lc. Cremoris, Lb. delbruckeii subsp. Lactis, Lb. delbruckeii subsp. bulgaricus, Lb. delbruckeii subsp. delbruckeii, Lb. Helveticus, Lb. casei.*

9. Starter culture for milk fermentation, comprising a microorganism of claims 1, 7 or 8.

10. Use of a plasmid of claims 3 and 6, alone or in combination with a conjugative plasmid, for conferring phage resistance to a bacterium.

## Patentansprüche

1. *Streptococcus thermophilus*-Stämme P-18383 und P-18384, hinterlegt bei BCCM/LMG (Gent/Belgien).

2. Nukleinsäuremolekül mit der Sequenz SEQ ID No. 1.

3. Plasmid, das das Nukleinsäuremolekül nach Anspruch 2 enthält.

4. Plasmid, erhältlich aus einer Kultur vom *Streptococcus thermophilus-Stamm* N. P-18384 nach Anspruch 1 mit 3375 Basenpaaren und je einer einzelnen Restriktionsschnittstelle für AccI-, AseI-, AsnI-, EcoRV-, FokI-, NotI-, PstI-, PvuII-, XbaI-Nuklease.

5. Plasmid, erhältlich aus einer Kultur vom *Streptococcus thermophilus-Stamm* N. P-18384 nach Anspruch 1 mit 6184 Basenpaaren und je einer einzelnen Restriktionsschnittstelle für AvaI-, HindIII-, KpnI-, PvuI-, SacI-, SacII-, SalI-, SpeI-, SphI-Nuklease.

6. Plasmid, erhältlich aus einer Kultur vom *Streptococcus thermophilus-Stamm* N. P-18383 nach Anspruch 1 mit 9515 Basenpaaren und je einer einzelnen Restriktionsschnittstelle für AvaI-, NotI-, PstI-, PvuI-, PvuII-, SacI-, SacII-, SalI-, SphI-Nuklease.

7. Mikroorganismus, der das Plasmid nach den Ansprüchen 3-6 enthält.

8. Mikroorganismus nach Anspruch 7, ausgewählt aus *Streptococcus thermophilus, Lactobacillus lactis, Bacillus subtilis, Escherichia coli, Lc. lactis subsp. lactis, Lc. lactis subsp. diacetylactis, Lc. Cremoris, Lb. delbruckeii subsp. Lactis, Lb. delbruckeii subsp. bulgaricus, Lb. delbruckeii subsp. delbruckeii, Lb. Helveticus, Lb. casei.*

9. Starterkultur zur Milchfermentation, umfassend einen Mikroorganismus nach Anspruch 1, 7 oder 8.

10. Verwendung eines Plasmids nach Anspruch 3 und 6, alleine oder in Kombination mit einem Konjugationsplasmid, zum Übertragen von Phagenresistenz auf ein Bakterium.

## Revendications

1. Souches de *Streptococcus thermophilus* déposées au BCCM/LMG (Gand/Belgique) P-18383 et P-18384.

2. Molécule d'acide nucléique ayant la séquence Séq. ID N° 1.

3. Plasmide contenant la molécule d'acide nucléique de la revendication 2.

4. Plasmide que l'on peut obtenir à partir d'une culture de la souche de *Streptococcus thermophilus* N. P-18384 selon la revendication 1, ayant 3375 paires de bases et un site de restriction unique pour chaque nucléase AccI, AseI, AsnI, EcoRV, FokI, NotI, PstI, PvulI, XbaI.

5. Plasmide que l'on peut obtenir à partir d'une culture de la souche de *Streptococcus thermophilus* N. P-18384 selon la revendication 1, ayant 6184 paires de bases et un site de restriction unique pour chaque nucléase AvaI, HindIII, KpnI, PvuI, SacI, SacII, SaII, SpeI, SphI.

6. Plasmide que l'on peut obtenir à partir d'une culture de la souche de *Streptococcus thermophilus* N. P-18383 selon la revendication 1, ayant 9515 paires de bases et un site de restriction unique pour chaque nucléase AvaI, NotI, PstI, PvuI, PvuII, SacI, SacII, SaII, SphI.

7. Microorganisme contenant le plasmide des revendications 3-6.

8. Microorganisme selon la revendication 7, choisi parmi *Streptococcus thermophilus, Lactobacillus lactis, Bacillus subtilis, Escherichia coli, Lc. lactis subsp. lactis, Lc. lactis subsp. diacetylactis, Lc. Cremoris, Lb. delbruckeii subsp. Lactis, Lb. delbruckeii subsp. bulgaricus, Lb. delbruckeii subsp. delbruckeii, Lb. Helveticus, Lb. casei.*

9. Ferment pour la fermentation lactique, comprenant un microorganisme des revendications 1, 7 ou 8.

10. Utilisation d'un plasmide des revendications 3 et 6, seul ou en combinaison avec un plasmide conjugué, pour conférer une résistance de phage à une bactérie.
